# EUROPÄISCHE PATENTSCHRIFT

(11) **EP 2 142 526 B1**
(45) Veröffentlichungstag und Bekanntmachung des Hinweises auf die Patenterteilung: **20.10.2010**
(21) Anmeldenummer: 08718289.5
(22) Anmeldetag: 27.03.2008
(51) Int. Cl.: C07D 323/06

(54) **VERFAHREN ZUR HERSTELLUNG VON CYCLISCHEN FORMALDEHYDDERIVATEN AUS POLYOXIDIALKYLETHERN**
METHOD FOR PRODUCING CYCLIC FORMALDEHYDE DERIVATIVES FROM POLYOXY DIALKYL ETHERS
PROCÉDÉ DE PRODUCTION DE DÉRIVÉS CYCLIQUES DE FORMALDÉHYDE À PARTIR D'ÉTHERS DE POLYOXYDIALKYLE

(30) Priorität: 30.03.2007 EP 07105348
(43) Veröffentlichungstag der Anmeldung: 13.01.2010
(73) Patentinhaber: BASF SE, 67056 Ludwigshafen (DE)
(72) Erfinder: STROEFER, Eckhard, 68163 Mannheim (DE); LANG, Neven, 68163 Mannheim (DE); SIEGERT, Markus, 69126 Heidelberg (DE); HASSE, Hans, 67661 Kaiserslautern (DE)
(86) Internationale Anmeldenummer: PCT/EP2008/053669
(87) Internationale Veröffentlichungsnummer: WO 2008/119743

(56) Entgegenhaltungen:
- DE-A1- 10 361 518

## Beschreibung

Trioxan wird in der Regel durch Destillation von wässriger Formaldehydlösung in Gegenwart saurer Katalysatoren hergestellt. Dem Formaldehyd und Wasser enthaltenden Destillat wird anschließend das Trioxan durch Extraktion mit halogenierten Kohlenwasserstoffen, wie Methylenchlorid oder 1,2-Dichlorethan, oder anderen, mit Wasser nicht mischbaren Lösungsmitteln entzogen.

DE-A 1 668 867 beschreibt ein Verfahren zur Abtrennung von Trioxan aus Wasser, Formaldehyd und Trioxan enthaltenden Gemischen durch Extraktion mit einem organischen Lösungsmittel. Dabei wird eine aus zwei Teilstrecken bestehende Extraktionsstrecke an einem Ende mit einem üblichen organischen, mit Wasser praktisch nicht mischbaren Extraktionsmittel für Trioxan beschickt, am anderen Ende mit Wasser. Zwischen den beiden Teilstrecken wird das zu trennende Destillat der Trioxan-Synthese zuführt. Auf der Seite der Lösungsmittelzuführung wird dann eine wässrige Formaldehydlösung und auf der Seite der Wasserzuführung eine praktisch formaldehydfreie Lösung von Trioxan in dem Lösungsmittel erhalten. In einem Beispiel wird das bei der Trioxan-Synthese entstandene Destillat aus 40 Gew.-% Wasser, 35 Gew.-% Trioxan und 25 Gew.-% Formaldehyd in den Mittelteil einer Pulsationskolonne eindosiert, am oberen Kolonnenende Methylenchlorid und am unteren Kolonnenende Wasser zugeführt. Dabei wird am unteren Kolonnenende eine etwa 25 gew.-%ige Lösung von Trioxan in Methylenchlorid und am oberen Kolonnenende eine etwa 30 gew.-%ige wässrige Formaldehydlösung erhalten.

Nachteil dieser Verfahrensweise ist der Anfall an Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrenstoffverordnung), deren Handhabung besondere Vorsichtsmaßnahmen erfordert.

DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in Trioxan und ein azeotropes Gemisch aus Trioxan, Wasser und Formaldehyd trennt. In dem Beispiel wird ein wässriges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trioxan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt. Nachteilig an dieser Verfahrensweise sind die sehr hohen Investitionen für die Pervaporationseinheit.

DE-A 103 61 518 beschreibt ein Verfahren zur Herstellung von Trioxan aus einer wässrigen Formaldehydlösung, bei dem ein Formaldehyd, Trioxan und Wasser enthaltender Einsatzstrom in einer vorgelagerten Trioxan-Synthesestufe aus einer wässrigen Formaldehydlösung hergestellt wird und anschließend aus diesem Strom Trioxan abgetrennt wird. Alternativ dazu können die Trioxansynthese und die erste Destillationsstufe der Trioxan-Abtrennung in einer Reaktivdestillation vereinigt werden.

Dazu wird in der Trioxan-Synthesestufe der Strom aus wässriger Formaldehydlösung in Gegenwart saurer homogen oder heterogen vorliegender Katalysatoren wie lonenaustauscherharze, Zeolithe, Schwefelsäure und p-Toluolsulfonsäure bei einer Temperatur von im Allgemeinen 70 bis 130 °C umgesetzt. Dabei kann in einer Destillationskolonne oder einem Verdampfer (Reaktivverdampfer) gearbeitet werden. Das Produktgemisch aus Trioxan/Formaldehyd und Wasser fällt dann als dampfförmiger Brüdenabzugsstrom des Verdampfers beziehungsweise als Kopfabzugsstrom am Kopf der Kolonne an. Die Trioxan-Synthesestufe kann auch in einem Festbett- oder Fließbettreaktor an einem heterogenen Katalysator, z. B. einem lonenaustauscherharz oder Zeolith, durchgeführt werden.

In einer weiteren Ausführungsform des in DE-A 103 61 518 beschriebenen Verfahrens werden die Trioxan-Synthesestufe und die erste Destillationsstufe als Reaktivdestillation in einer Reaktionskolonne durchgeführt. Diese kann im Abtriebsteil ein Katalysator-Festbett aus einem heterogenen sauren Katalysator enthalten. Alternativ kann die Reaktivdestillation auch in Gegenwart eines homogenen Katalysators durchgeführt werden, wobei der saure Katalysator zusammen mit der wässrigen Formaldehyd-Lösung im Kolonnensumpf vorliegt.

Allen im Stand der Technik beschriebenen Verfahren ist gemeinsam, dass Trioxan sauer katalysiert aus wässrigen Formaldehydlösungen hergestellt wird. Als problematisch erweist sich dabei, dass Trioxan, Formaldehyd und Wasser ein ternäres Azeotrop bilden, welches bei einem Druck von 1 bar die Zusammensetzung 69,5 Gew.-% Trioxan, 5,4 Gew.-% Formaldehyd und 25,1 Gew.-% Wasser aufweist. Daher ist die Abtrennung von reinem Trioxan aus dem Formaldehyd und Wasser enthaltenden Produktgemisch der Trioxan-Synthese schwierig. Gemäß DE-A 103 61 518 wird dieses Azeotrop durch Druckwechseldestillation umgangen, bei dem eine erste und eine zweite Destillation bei verschiedenen Drücken durchgeführt werden. In einer ersten Destillationskolonne, welche bei niedrigerem Druck betrieben wird, wird das Ausgangsgemisch in ein Trioxan/Wasser-Gemisch mit geringem Formaldehyd-Gehalt und ein im Wesentlichen trioxanfreies Formaldehyd/Wasser-Gemisch aufgetrennt. Das trioxanfreie Formaldehyd/Wasser-Gemisch kann in die Trioxan-Synthese zurückgeführt werden. In einer zweiten, bei höherem Druck betriebenen Destillationskolonne wird das Trioxan/Formaldehyd/Wasser-Gemisch in reines Trioxan und ein Trioxan/Formaldehyd/Wasser-Gemisch mit niedrigerem Trioxangehalt aufgetrennt.

Aufgabe der Erfindung ist es, ein weiteres, vorteilhaftes Verfahren zur Herstellung von Trioxan bereitzustellen. Aufgabe der Erfindung ist es insbesondere, ein vorteilhaftes Verfahren zur Herstellung von Trioxan bereitzustellen, bei dem keine schwer aufzutrennenden Formaldehyd/Trioxan/Wasser-Azeotrope gebildet werden.

Gelöst wird die Aufgabe durch ein Verfahren zur Herstellung von Trioxan aus Trioxymethylenglykoldimethylether (POMDMEₙ₌₃) durch Umsetzung von Trioxymethylenglykoldimethylether in Gegenwart eines sauren Katalysators und anschließender destillativer Aufarbeitung des Reaktionsgemischs mit den Schritten:
a) Einspeisung von Trioxymethylenglykoldimethylether (POMDMEₙ₌₃) oder eines Trioxymethylenglykoldimethylether enthaltenden Gemischs in einen Reaktor und Umsetzung in Gegenwart eines sauren Katalysators zu einem Gemisch a enthaltend Trioxan, Formaldehyd, Wasser, Methylenglykol (MG), Polyoxymethylenglykole (MG_{n>1}), Methanol, Hemiformale (HF), Methylal (POMDMEₙ₌₁) und Polyoxymethylenglykoldimethylether (POMDME_{n>1});
b) destillative Auftrennung des Reaktionsgemischs a in eine Leichtsiederfraktion b1 enthaltend Formaldehyd, Wasser, Methylenglykol, Methanol, Hemiformal (HFₙ₌₁), Methylal und Dioxymethylenglykoldimethylether (POMDMEₙ₌₂) und eine Schwersiederfraktion b2 enthaltend Trioxan, Polyoxymethylenglykole (MG_{n>1}), Hemiformale (HF_{n>1}) und Polyoxymethylenglykoldimethylether (POMDME_{n>2});
c) destillative Auftrennung der Schwersiederfraktion b2 in eine Leichtsiederfraktion c1 enthaltend Trioxan und eine Schwersiederfraktion c2 enthaltend Polyoxymethylenglykole (MG_{n>1}), Hemiformale (HF_{n>1}) und Polyoxymethylenglykoldimethylether (POMDME_{n>2}).

In Schritt a) wird Trioxymethylenglykoldimethylether (POMDMEₙ₌₃) oder ein Trioxymethylenglykoldimethylether enthaltendes Gemisch in Gegenwart eines sauren Katalysators umgesetzt. Der dabei eingesetzte saure Katalysator kann ein homogener oder heterogener saurer Katalysator sein. Im Allgemeinen wird die Umsetzung in Gegenwart geringer Mengen Wasser durchgeführt. Geeignete saure Katalysatoren sind allgemein Säuren mit einem pKs-Wert von < 4, Mineralsäuren wie Phosphorsäure, Schwefelsäure, Sulfonsäuren wie Trifluormethansulfonsäure und para-Toluolsulfonsäure, Heteropolysäuren, saure lonenaustauscherharze, Zeolithe, Aluminosilikate, Siliciumdioxid, Aluminiumoxid, Titandioxid und Zirkondioxid. Oxidische Katalysatoren können, um deren Säurestärke zu erhöhen, mit Sulfat- oder Phosphat-Gruppen dotiert sein, im Allgemeinen in Mengen von 0,05 bis 10 Gew.-%. Die Umsetzung kann in einem Rührkesselreaktor (CSTR) oder einem Rohrreaktor durchgeführt werden. Wird ein heterogener Katalysator eingesetzt, ist ein Festbettreaktor bevorzugt. Neben Trioxan kann in geringen Mengen auch Tetraoxan gebildet werden.

In Schritt b) wird das Produktgemisch a) - vorzugsweise in einer ersten Destillationskolonne - in eine Leichtsiederfraktion b1 enthaltend Formaldehyd, Wasser, Methylenglykol, Methanol, Hemiformal (HFₙ₌₁), Methylal und Dioxymethylenglykoldimethylether (POMDMEₙ₌₂) und eine Schwersiederfraktion b2 enthaltend Trioxan, Polyoxymethylenglykole (MG_{n>1}), Hemiformale (HF_{n>1}) und Polyoxymethylenglykoldimethylether mit 3 oder mehr Oxymethyleneinheiten (POMDME_{n>2}) aufgetrennt. Gegebenenfalls gebildetes Tetraoxan wird zusammen mit Trioxan in der Schwersiederfraktion b2 abgetrennt, kann aber auch zu einem gewissen Anteil in der Leichtsiederfraktion b1 enthalten sein. Die Leichtsiederfraktion b1 kann darüber hinaus in geringen Mengen noch weitere Nebenkomponenten wie Ameisensäure und Methylformiat enthalten.

Der Index n bezeichnet jeweils die Zahl der Oxymethylen-Einheiten. Als Hemiformal wird das Formaldehyd/Methanol-Halbacetat bezeichnet. Hemiformale HF_{n>1} sind die höheren Homologen des Formaldehyd-Halbacetats mit n CH₂O-Einheiten.

Die in den nachfolgend beschriebenen Schritten eingesetzten Destillationskolonnen sind Kolonnen üblicher Bauart. In Frage kommen Füllkörperkolonnen, Bodenkolonnen und Packungskolonnen, bevorzugt sind Bodenkolonnen und Packungskolonnen. Der Begriff "Leichtsiederfraktion" wird für das im oberen Teil, der Begriff "Schwersiederfraktion" für das im unteren Teil der Kolonne entnommene Gemisch verwendet. Im Allgemeinen wird die Leichtsiederfraktion am Kolonnenkopf, die Schwersiederfraktion am Kolonnensumpf entnommen. Dies ist jedoch nicht zwingend. Möglich ist auch die Entnahme über Seitenabzüge im Abtriebs- beziehungsweise Verstärkungsteil der Kolonne.

Die erste Destillationskolonne weist im Allgemeinen eine Stufenzahl von 1 bis 50, vorzugsweise von 3 bis 30 auf. Sie wird bei einem Druck von im Allgemeinen 1 bis 5 bar, vorzugsweise von 1 bis 3 bar, betrieben. Die Kopftemperatur beträgt im Allgemeinen 0 bis 150 °C, vorzugsweise 20 bis 120 °C, die Sumpftemperatur beträgt im Allgemeinen 70 bis 220 °C, vorzugsweise 80 bis 190 °C.

Vorzugsweise wird die Schwersiederfraktion b2 in den Reaktor des Schrittes a) zurückgeführt.

Die Leichtsiederfraktion b1 wird anschließend - vorzugsweise in einer zweiten Destillationskolonne - in eine Leichtsiederfraktion c1 enthaltend Trioxan und eine Schwersiederfraktion c2 enthaltend die übrigen Komponenten der Fraktion b1 aufgetrennt. Gegebenenfalls enthaltenes Tetraoxan wird zusammen mit dem Trioxan abgetrennt.

Die zweite Destillationskolonne weist im Allgemeinen eine Stufenzahl von 1 bis 50, vorzugsweise von 3 bis 30 auf. Sie wird bei einem Druck von 0,5 bis 5 bar, vorzugsweise von 0,8 bis 3 bar, betrieben. Die Kopftemperatur beträgt im Allgemeinen 0 bis 140 °C, vorzugsweise 20 bis 110 °C, die Sumpftemperatur beträgt im Allgemeinen 80 bis 220 °C, vorzugsweise 90 bis 200 °C.

In einer Variante des erfindungsgemäßen Verfahrens werden von der Leichtsiederfraktion b1 Methanol und Methylformiat abgetrennt. Dies kann in einer Leichtsieder-Abtrennstufe geschehen, wobei als weitere Leichtsieder noch Methylal und Hemiformal mit abgetrennt werden. Die Leichtsiederfraktion b1 wird also in eine Fraktion d1 enthaltend Wasser, Methylenglykol, Methanol, Methylformiat und Hemiformal (HFₙ₌₁) und eine Fraktion d2 enthaltend Formaldehyd, Wasser und Dioxymethylenglykoldimethylether (POMDMEₙ₌₂) aufgetrennt. Beide Fraktionen d1 und d2 können daneben noch Ameisensäure enthalten. Die Fraktion d2 wird in den Trioxan-Synthesereaktor (Schritt a)) zurückgeführt.

Der Trioxymethylenglykoldimethylether (POMDMEₙ₌₃) oder das diesen enthaltende Gemisch können in einer vorgelagerten Synthese durch Umsetzung eines Formaldehyd und Methanol enthaltenden Gemischs und anschließender destillativer Aufarbeitung des Produktgemischs erhalten werden.

In einer Variante des erfindungsgemäßen Verfahrens wird die gesamte Leichtsiederfraktion b1 enthaltend Formaldehyd, Wasser, Methylenglykol, Methanol, Hemiformal (HFₙ₌₁), Methylal und Dioxymethylenglykoldimethylether (POMDMEₙ₌₂) ohne weitere Auftrennung in die Trioxymethylenglykoldimethylether-Synthese zurückgeführt.

In einer weiteren Variante des erfindungsgemäßen Verfahrens wird die Leichtsiederfraktion b1, wie oben beschrieben, in eine Leichtsiederfraktion d1 enthaltend Wasser, Methylal, Methylenglykol, Methanol und Hemiformal (HFₙ₌₁) und eine Schwersiederfraktion d2 enthaltend Formaldehyd, Wasser und Dioxymethylenglykoldimethylether (POMDMEₙ₌₂) aufgetrennt, die Fraktion d1 in den Trioxan-Synthesereaktor (Schritt a)) und die Fraktion d2 in die Trioxymethylendimethylether-Synthese zurückgeführt.

Alternativ kann die Fraktion d2 auch als Nebenprodukt aus dem Verfahren ausgeschleust werden oder in eine der POMDMEₙ₌₃-Synthese vorgelagerte Formaldehyd-Synthese geführt werden.

In einer bevorzugten Ausführungsform wird in der Trioxan-Synthese (Schritt a)) ein Gemisch enthaltend Tri- und Tetraoxymethylenglykoldimethylether (POMDME_{n=3,4}) eingesetzt. Dieses wird bevorzugt nach einem der nachstehend beschriebenen Verfahren erhalten.

In jüngerer Zeit haben Polyoxymethylendimethylether als Dieselkraftstoff-Additive Bedeutung erlangt. Zur Verringerung der Rauch- und Rußbildung bei der Verbrennung von Dieselkraftstoff werden diesem Polyoxymethylendimethylether als sauerstoffhaltige Verbindungen, welche nur wenige oder überhaupt keine C-C-Bindungen aufweisen, zugesetzt. Hierbei haben sich die POMDME_{n=3,4} als besonders wirksam erwiesen. Werden Gemische enthaltend Tri- und Tetraoxymethylenglykoldimethylether (POMDME_{n=3,4}) aber in großen Mengen hergestellt, um als Dieselkraftstoff-Additive Verwendung zu finden, dann lässt sich ausgehend von diesen Gemischen ein sehr wirtschaftliches Verfahren der Trioxan-Herstellung realisieren, da in diesem Fall von der "Economy of Scale" der POMDME-Synthese profitiert würde. In diesem Fall würde also ein Teilstrom des produzierten POMDME_{n=3,4} zu Trioxan weiterverarbeitet.

Wird die Leichtsiederfraktion b1 in eine Fraktion d1 enthaltend Wasser, Methylal, Methylenglykol, Methanol, Methylformiat und Hemiformal (HFₙ₌₁) und eine Fraktion d2 enthaltend Formaldehyd, Wasser und Dioxymethylenglykoldimethylether (POMDMEₙ₌₂) aufgetrennt und die Leichtsiederfraktion d1 in den Trioxan-Synthesereaktor (Schritt a)) zurückgeführt, dann wird, wenn Tri- und Tetraoxymethylenglykoldimethylether (POMDME_{n=3,4}) nach den unten beschriebenen Verfahrensvarianten erhalten werden, die Schwersiederfraktion d2 bevorzugt in den Schritt A) der nachfolgend beschriebenen Synthesevarianten zurückgeführt.

Gemäß einer ersten Variante wird ein Gemisch aus Tri- und Tetraoxymethylenglykoldimethylether (POMDME_{n=3,4}) hergestellt durch Umsetzung von Formaldehyd mit Methanol und anschließender destillativer Aufarbeitung des Reaktionsgemischs mit den Schritten:
A) Einspeisung von wässriger Formaldehydlösung und von Methanol in einen Reaktor und Umsetzung zu einem Gemisch A enthaltend Formaldehyd, Wasser, Methylenglykol (MG), Polyoxymethylenglykole (MG_{n>1}), Methanol, Hemiformale (HF), Methylal (POMDMEₙ₌₁) und Polyoxymethylenglykoldimethylether (POMDME_{n>1});
B) Einspeisung des Reaktionsgemischs A in eine erste Destillationskolonne und Auftrennung in eine Leichtsiederfraktion B1 enthaltend Formaldehyd, Wasser, Methylenglykol, Methanol, Methylal und Dioxymethylenglykoldimethylether (POMDMEₙ₌₂) und eine Schwersiederfraktion B2 enthaltend Formaldehyd, Wasser, Methanol, Polyoxymethylenglykole, Hemiformale und Polyoxymethylenglykoldimethylether (POMDME_{n>1});
C) Einspeisung der Schwersiederfraktion B2 in eine zweite Destillationskolonne und Auftrennung in eine Leichtsiederfraktion C1 enthaltend Formaldehyd, Wasser, Methylenglykol, Polyoxymethylenglykole, Methanol, Hemiformale, Di-, Tri- und Tetraoxymethylenglykoldimethylether (POMDME_{n=2,3,4}) und eine Schwersiederfraktion C2 enthaltend Polyoxymethylenglykole, schwersiedende Hemiformale (HF_{n>1}) und schwersiedende Polyoxymethylenglykoldimethylether (POMDME_{n>4});
D) Einspeisung der Leichtsiederfraktion C1 und gegebenenfalls eines oder mehrerer Rückführströme aus Formaldehyd, Wasser, Methylenglykol und Polyoxymethylenglykolen in eine dritte Destillationskolonne und Auftrennung in eine Leichtsiederfraktion d1 enthaltend Formaldehyd, Wasser, Methanol, Polyoxymethylenglykole, Hemiformale und Dioxymethylenglykoldimethylether (POMDMEₙ₌₂) und eine Schwersiederfraktion D2 im Wesentlichen bestehend aus Formaldehyd, Wasser, Methylenglykol, Polyoxymethylenglykolen, Tri- und Tetraoxymethylenglykoldimethylether (POMDME_{n=3,4});
E) Einspeisung der Schwersiederfraktion D2 in einen Phasentrennapparat und Auftrennung in eine wässrige Phase E1 im Wesentlichen bestehend aus Formaldehyd, Wasser, Methylenglykol und Polyoxymethylenglykolen und eine organische Phase E2 enthaltend Tri- und Tetraoxymethylenglykoldimethylether (POMDME_{n=3,4});
F) Einspeisung der organischen Phase E2 in eine vierte Destillationskolonne und Auftrennung in eine Leichtsiederfraktion F1 im Wesentlichen bestehend aus Formaldehyd, Wasser, Methylenglykol und Polyoxymethylenglykolen und eine Schwersiederfraktion F2 im Wesentlichen bestehend aus Tri- und Tetraoxymethylenglykoldimethylether (POMDME_{n=3,4});
G) optional Einspeisung der wässrigen Phase E1 in eine fünfte Destillationskolonne und Auftrennung in eine Leichtsiederfraktion G1 im Wesentlichen bestehend aus Formaldehyd, Wasser, Methylenglykol und Polyoxymethylenglykolen und eine Schwersiederfraktion im Wesentlichen bestehend aus Wasser.

In einem Schritt A) werden wässrige Formaldehydlösung und Methanol in einen Reaktor eingespeist und zu einem Gemisch a enthaltend Formaldehyd, Wasser, Methylenglykol, Polyoxymethylenglykole, Methanol, Hemiformale, Methylal und Polyoxymethylenglykoldimethylether umgesetzt.

In Schritt A) kann handelsübliche wässrige Formaldehydlösung direkt eingesetzt werden oder diese kann zuvor aufkonzentriert werden, beispielsweise wie in EP-A 1 063 221 beschrieben. Im Allgemeinen beträgt die Formaldehydkonzentration der wässrigen Formaldehydlösung von 20 bis 60 Gew.-%. Methanol wird vorzugsweise in reiner Form eingesetzt. Die Gegenwart von geringen Mengen anderer Alkohole wie Ethanol ist nicht störend. Möglich ist die Verwendung von Methanol, das bis zu 30 Gew.-% Ethanol enthält.

Wasser, monomerer (freier) Formaldehyd, Methylenglykol (MG) und oligomere Polyoxymethylenglykole unterschiedlicher Kettenlänge (MG_{n>1}) liegen in wässrigen Lösungen nebeneinander in einem thermodynamischen Gleichgewicht vor, das durch eine bestimmte Verteilung der Polyoxymethylenglykole unterschiedlicher Länge gekennzeichnet ist. Der Begriff "wässrige Formaldehydlösung" bezieht sich dabei auch auf Formaldehydlösungen, die praktisch kein freies Wasser, sondern im Wesentlichen nur noch in Form von Methylenglykol beziehungsweise in den endständigen OH-Gruppen der Polyoxymethylenglykole chemisch gebundenes Wasser enthalten. Dies ist insbesondere bei konzentrierten Formaldehydlösungen der Fall. Polyoxymethylenglykole können dabei beispielsweise zwei bis neun Oxymethyleneinheiten aufweisen.

Der dabei eingesetzte saure Katalysator kann ein homogener oder heterogener saurer Katalysator sein. Geeignete saure Katalysatoren sind Mineralsäuren wie weitgehend wasserfreie Schwefelsäure, Sulfonsäuren wie Trifluormethansulfonsäure und para-Toluolsulfonsäure, Heteropolysäuren, saure lonenaustauscherharze, Zeolithe, Aluminosilikate, Siliciumdioxid, Aluminiumoxid, Titandioxid und Zirkondioxid. Oxidische Katalysatoren können, um deren Säurestärke zu erhöhen, mit Sulfat- oder Phosphat-Gruppen dotiert sein, im Allgemeinen in Mengen von 0,05 bis 10 Gew.-%. Die Umsetzung kann in einem Rührkesselreaktor (CSTR) oder einem Rohrreaktor durchgeführt werden. Wird ein heterogener Katalysator eingesetzt, ist ein Festbettreaktor bevorzugt. Wird ein Katalysator-Festbett verwendet, kann das Produktgemisch anschließend mit einem Anionenaustauscherharz in Kontakt gebracht werden, um ein im Wesentlichen säurefreies Produktgemisch zu erhalten. Im weniger vorteilhaften Fall kann auch eine Reaktivdestillation eingesetzt werden.

Die Umsetzung erfolgt im Allgemeinen bei einer Temperatur von 0 bis 200 °C, bevorzugt 50 bis 150 °C, und einem Druck von 1 bis 20 bar, bevorzugt 2 bis 10 bar.

In einem Schritt B) wird das Reaktionsgemisch A in eine erste Destillationskolonne eingespeist und in eine Leichtsiederfraktion B1 enthaltend Formaldehyd, Wasser, Methylenglykol, Methanol, Methylal und Dioxymethylenglykoldimethylether (POMDMEₙ₌₂) und eine Schwersiederfraktion B2 enthaltend Formaldehyd, Wasser, Methanol, Polyoxymethylenglykole, Hemiformale und Polyoxymethylenglykoldimethylether (POMDME_{n>1}) aufgetrennt.

Die erste Destillationskolonne weist im Allgemeinen eine Stufenzahl von 3 bis 50, vorzugsweise von 5 bis 20 auf. Sie wird bei einem Druck von 0,2 bis 10 bar, vorzugsweise von 0,8 bis 6 bar, betrieben. Die Kopftemperatur beträgt im Allgemeinen -20 bis +160 °C, vorzugsweise + 20 bis 130 °C, die Sumpftemperatur beträgt im Allgemeinen +30 bis +320 °C, vorzugsweise +90 bis +200 °C.

Im Allgemeinen wird die Leichtsiederfraktion B1 in den POMDME-Reaktor (Schritt A)) zurückgeführt.

In einem Schritt C) wird die Schwersiederfraktion B2 in eine zweite Destillationskolonne eingespeist und in eine Leichtsiederfraktion C1 enthaltend Formaldehyd, Wasser, Methylenglykol, Polyoxymethylenglykole, Methanol, Hemiformale, Di-, Tri- und Tetraoxymethylenglykoldimethylether (POMDME_{n=2,3,4}) und eine Schwersiederfraktion C2 enthaltend Polyoxymethylenglykole, schwersiedende Hemiformale (HF_{n>1}) und schwersiedende Polyoxymethylenglykoldimethylether (POMDME_{n>4}) aufgetrennt.

Die zweite Destillationskolonne weist im Allgemeinen eine Stufenzahl von 3 bis 50, vorzugsweise von 5 bis 20 auf. Sie wird bei einem Druck von 0,1 bis 10 bar, vorzugsweise von 0,2 bis 6 bar, betrieben. Die Kopftemperatur beträgt im Allgemeinen +20 bis +260 °C, vorzugsweise +20 bis +230 °C, die Sumpftemperatur beträgt im Allgemeinen +80 bis +320 °C, vorzugsweise +100 bis +250 °C.

Die Schwersiederfraktion kann in den POMDME-Reaktor (Schritt A)) zurückgeführt werden.

In einer Ausführungsform wird die Schwersiederfraktion C2 zusammen mit Methanol in einen weiteren (zweiten) Reaktor eingespeist und umgesetzt. Dabei werden langkettige oligomere Polyoxymethylenglykole, Hemiformale und Polyoxymethylenglykoldimethylether durch Umsetzung mit Methanol in kürzere Ketten gespalten. Dabei können die gleichen sauren Katalysatoren wie im ersten Reaktor eingesetzt werden. Das Reaktionsprodukt wird vorzugsweise in den (ersten) Reaktor (des Schrittes A)) eingespeist. Das Reaktionsprodukt kann auch direkt in die erste Destillationskolonne eingespeist werden. Die Temperatur in dem zweiten Reaktor ist im Allgemeinen höher als in dem ersten Reaktor und beträgt im Allgemeinen 50 bis 320 °C, vorzugsweise 80 bis 250 °C. Der zweite Reaktor wird dabei bei einem Druck von im Allgemeinen 1 bis 20 bar, vorzugsweise 2 bis 10 bar betrieben.

In einem weiteren Schritt D) werden die Leichtsiederfraktion C1 und gegebenenfalls ein oder mehrere Rückführströme aus Formaldehyd, Wasser, Methylenglykol und Polyoxymethylenglykolen in eine dritte Destillationskolonne eingespeist und in eine Leichtsiederfraktion D1 enthaltend Formaldehyd, Wasser, Methanol, Polyoxymethylenglykole, Hemiformale und Dioxymethylenglykoldimethylether (POMDMEₙ₌₂) und eine Schwersiederfraktion D2 im Wesentlichen bestehend aus Formaldehyd, Wasser, Methylenglykol, Polyoxymethylenglykolen, Tri- und Tetraoxymethylenglykoldimethylether (POMDME_{n=3,4}) aufgetrennt.

"Im Wesentlichen bestehend aus" hat hier und nachfolgend die Bedeutung, dass die betreffende Fraktion zu mindestens 90 Gew.-%, vorzugsweise zu mindestens 95 Gew.-% aus den genannten Komponenten besteht. Die Schwersiederfraktion D2 enthält insbesondere praktisch keinen Dioxymethylenglykoldimethylether mehr. Dessen Gehalt in der Schwersiederfraktion D2 beträgt im Allgemeinen < 3 Gew.-%.

Die dritte Destillationskolonne weist im Allgemeinen eine Stufenzahl von 1 bis 50, vorzugsweise von 1 bis 20 auf. Sie wird bei einem Druck von 0,1 bis 10 bar, vorzugsweise von 0,2 bis 6 bar, betrieben. Die Kopftemperatur beträgt im Allgemeinen 0 bis +160 °C, vorzugsweise +20 bis +130 °C, die Sumpftemperatur beträgt im Allgemeinen +50 bis +260 °C, vorzugsweise +80 bis +220 °C.

Im Allgemeinen wird die Leichtsiederfraktion D1 in den POMDME-Reaktor (Schritt A)) zurückgeführt.

In einem Schritt E) wird die Schwersiederfraktion D2 in einen Phasentrennapparat eingespeist und in eine wässrige Phase E1 im Wesentlichen bestehend aus Formaldehyd, Wasser, Methylenglykol und Polyoxymethylenglykolen und eine organische Phase E2 enthaltend Tri- und Tetraoxymethylenglykoldimethylether (POMDME_{n=3,4}) aufgetrennt. Die organische Phase E2 enthält daneben ebenfalls noch Formaldehyd, Wasser, Methylenglykol und Polyoxymethylenglykole.

In einem Schritt F) wird die organische Phase E2 in eine vierte Destillationskolonne eingespeist und in eine Leichtsiederfraktion F1 im Wesentlichen bestehend aus Formaldehyd, Wasser, Methylenglykol und Polyoxymethylenglykolen und eine Schwersiederfraktion F2 im Wesentlichen bestehend aus Tri- und Tetraoxymethylenglykoldimethylether (POMDME_{n=3,4}) aufgetrennt.

Die vierte Destillationskolonne weist im Allgemeinen eine Stufenzahl von 1 bis 100, vorzugsweise von 1 bis 50 auf. Sie wird bei einem Druck von 0,1 bis 10 bar, vorzugsweise von 0,2 bis 6 bar, betrieben. Die Kopftemperatur beträgt im Allgemeinen 0 bis +160 °C, vorzugsweise +20 bis +130 °C, die Sumpftemperatur beträgt im Allgemeinen +100 bis +260 °C, vorzugsweise +150 bis +240 °C.

Die Schwersiederfraktion F2 stellt das Wertprodukt dar. Sie kann mehr als 99 Gew.-% POMDME_{n=3,4} enthalten.

Im Allgemeinen wird in einem weiteren (optionalen) Schritt G) die wässrige Phase E1 weiter aufgearbeitet. Dazu wird diese in eine fünfte Destillationskolonne eingespeist und in eine Leichtsiederfraktion G1 im Wesentlichen bestehend aus Formaldehyd, Wasser, Methylenglykol und Polyoxymethylenglykolen und eine Schwersiederfraktion im Wesentlichen bestehend aus Wasser aufgetrennt.

Die fünfte Destillationskolonne weist im Allgemeinen eine Stufenzahl von 1 bis 30, vorzugsweise von 1 bis 20 auf. Sie wird bei einem Druck von 0,1 bis 10 bar, vorzugsweise von 0,2 bis 6 bar, betrieben. Die Kopftemperatur beträgt im Allgemeinen -20 bis +120 °C, vorzugsweise +20 bis +100 °C, die Sumpftemperatur beträgt im Allgemeinen +40 bis +180 °C, vorzugsweise +60 bis + 150 °C.

Die Leichtsiederfraktionen F1 und/oder G1 können als Rückführströme in die dritte Destillationskolonne (Schritt D)) zurückgeführt werden. Bevorzugt werden sie in die dritte Destillationskolonne zurückgeführt. Die Leichtsiederfraktionen F1 und/oder G1 können aber auch als Rückführströme in den POMDME-Reaktor (Schritt A)) zurückgeführt werden.

In einer zweiten, alternativen Verfahrensvariante wird ein Gemisch aus Tri- und Tetraoxymethylenglykoldimethylether (POMDME_{n=3,4}) hergestellt durch Umsetzung von Formaldehyd mit Methanol und anschließender destillativer Aufarbeitung des Reaktionsgemischs mit den Schritten:
A) Einspeisung von wässriger Formaldehydlösung und von Methanol in einen Reaktor und Umsetzung zu einem Gemisch A enthaltend Formaldehyd, Wasser, Methylenglykol (MG), Polyoxymethylenglykole (MG_{n>1}), Methanol, Hemiformale (HF), Methylal (POMDMEₙ₌₁) und Polyoxymethylenglykoldimethylether (POMDME_{n>1});
B) Einspeisung des Reaktionsgemischs A in einen Reaktivverdampfer und Auftrennung in eine Leichtsiederfraktion B1 enthaltend Formaldehyd, Wasser, Methanol, Methylenglykol, Polyoxymethylenglykole, Hemiformale, Methylal und Polyoxymethylenglykoldimethylether (POMDME_{n>1}) und eine Schwersiederfraktion B2 enthaltend Polyoxymethylenglykole, schwersiedende Hemiformale (HF_{n>1}) und schwersiedende Polyoxymethylenglykoldimethylether (POMDME_{n>4}) und Rückführung der Schwersiederfraktion B2 in den Reaktor (Schritt a));
C) Einspeisung der Leichtsiederfraktion B1 in eine erste Destillationskolonne und Auftrennung in eine Leichtsiederfraktion C1 enthaltend Formaldehyd, Wasser, Methylenglykol, Methanol, Hemiformale, Methylal, Di-, Tri- und Tetraoxymethylenglykoldimethylether (POMDME_{n=2,3,4}) und eine Schwersiederfraktion C2 enthaltend Polyoxymethylenglykole, schwersiedende Hemiformale (HF_{n>1}) und schwersiedende Polyoxymethylenglykoldimethylether (POMDME_{n>4}) und Rückführung der Schwersiederfraktion C2 in den Reaktivverdampfer (Schritt A));
D) Einspeisung der Leichtsiederfraktion C1 in eine zweite Destillationskolonne und Auftrennung in eine Leichtsiederfraktion D1 enthaltend Formaldehyd, Wasser, Methanol, Polyoxymethylenglykole, Hemiformale, Methylal und Dioxymethylenglykoldimethylether (POMDMEₙ₌₂) und eine Schwersiederfraktion D2 im Wesentlichen bestehend aus Formaldehyd, Wasser, Methylenglykol, Polyoxymethylenglykolen, Tri- und Tetraoxymethylenglykoldimethylether (POMDME_{n=3,4});
E) Einspeisung der Schwersiederfraktion D2 in einen Phasentrennapparat und Auftrennung in eine wässrige Phase E1 im Wesentlichen bestehend aus Formaldehyd, Wasser, Methylenglykol und Polyoxymethylenglykolen und eine organische Phase E2 enthaltend Tri- und Tetraoxymethylenglykoldimethylether (POMDME_{n=3,4});
F) Einspeisung der organischen Phase E2 in eine dritte Destillationskolonne und Auftrennung in eine Leichtsiederfraktion F1 im Wesentlichen bestehend aus Formaldehyd, Wasser, Methylenglykol und Polyoxymethylenglykolen und eine Schwersiederfraktion F2 im Wesentlichen bestehend aus Tri- und Tetraoxymethylenglykoldimethylether (POMDME_{n=3,4});
G) optional Einspeisung der wässrigen Phase E1 in eine vierte Destillationskolonne und Auftrennung in eine Leichtsiederfraktion G1 im Wesentlichen bestehend aus Formaldehyd, Wasser, Methylenglykol und Polyoxymethylenglykolen und eine Schwersiederfraktion im Wesentlichen bestehend aus Wasser.

Abweichend von der ersten Variante wird in dem Schritt B) das Reaktionsgemisch A in einen Reaktivverdampfer eingespeist und in eine Leichtsiederfraktion B1 enthaltend Formaldehyd, Wasser, Methanol, Methylenglykol, Polyoxymethylenglykole, Hemiformale, Methylal und Polyoxymethylenglykoldimethylether (POMDME_{n>1}) und eine Schwersiederfraktion B2 enthaltend Polyoxymethylenglykole, Hemiformale (HF_{n>1}) und Polyoxymethylenglykole (POMDME_{n>3}) aufgetrennt. Die Schwersiederfraktion B2 wird in den Reaktor (Schritt A)) zurückgeführt.

Der Reaktiwerdampfer stellt den Sumpfverdampfer der ersten Destillationskolonne dar. Die aus der ersten Destillationskolonne zurücklaufende Fraktion C2 enthält Polyoxymethylenglykole, schwersiedende Hemiformale (HF_{n>1}) und schwersiedende Polyoxymethylenglykole (POMDME_{n>4}). Diese Fraktion vermischt sich in dem Reaktivverdampfer mit dem Reaktionsgemisch A, welches einen höheren Anteil an Wasser, Methanol, Polyoxymethylenglykolen, Hemiformalen und Polyoxymethylenglykoldimethylether kürzerer Kettenlänge enthält. So kommt es in dem Reaktivverdampfer zur Spaltung langkettiger Komponenten in Komponenten kürzerer Kettenlänge. Der Reaktivverdampfer wird im Allgemeinen bei dem Druck der ersten Kolonne betrieben. Er kann jedoch auch bei höherem Druck betrieben werden. Der Betriebsdruck des Reaktivverdampfers liegt im Allgemeinen bei 0,1 bis 20 bar, vorzugsweise 0,2 bis 10 bar, die Betriebstemperatur im Allgemeinen bei 50 bis 320 °C, vorzugsweise bei 80 bis 250 °C.

In einem Schritt C) wird die Leichtsiederfraktion B1 in eine erste Destillationskolonne eingespeist und in eine Leichtsiederfraktion C1 enthaltend Formaldehyd, Wasser, Methylenglykol, Methanol, Hemiformale, Methylal, Di-, Tri- und Tetraoxymethylenglykoldimethylether (POMDME_{n=2,3,4}) und eine Schwersiederfraktion C2 enthaltend Polyoxymethylenglykole, schwersiedende Hemiformale (HF_{n>1}) und schwersiedende Polyoxymethylenglykoldimethylether (POMDME_{n>4}) aufgetrennt. Die Schwersiederfraktion C2 wird in den Reaktivverdampfer (Schritt B) zurückgeführt.

Die erste Destillationskolonne weist im Allgemeinen eine Stufenzahl von 2 bis 50, vorzugsweise von 5 bis 20 auf. Sie wird bei einem Druck von 0,1 bis 10 bar, vorzugsweise von 0,2 bis 6 bar, betrieben. Die Kopftemperatur beträgt im Allgemeinen 0 bis 260 °C, vorzugsweise 20 bis 230 °C, die Sumpftemperatur beträgt der Temperatur des Reaktivverdampfers.

In einem Schritt D) wird die Leichtsiederfraktion C1 in eine zweite Destillationskolonne eingespeist und in eine Leichtsiederfraktion D1 enthaltend Formaldehyd, Wasser, Methanol, Polyoxymethylenglykole, Hemiformale, Methylal und Dioxymethylenglykoldimethylether (POMDMEₙ₌₂) und eine Schwersiederfraktion D2 im Wesentlichen bestehend aus Formaldehyd, Wasser, Methylenglykol, Polyoxymethylenglykolen, Tri- und Tetraoxymethylenglykoldimethylether (POMDME_{n=3,4}) aufgetrennt.

Die zweite Destillationskolonne weist im Allgemeinen eine Stufenzahl von 1 bis 50, vorzugsweise von 1 bis 20 auf. Sie wird bei einem Druck von 0,1 bis 10 bar, vorzugsweise von 0,2 bis 6 bar, betrieben. Die Kopftemperatur beträgt im Allgemeinen 0 bis 160 °C, vorzugsweise 20 bis 130 °C, die Sumpftemperatur beträgt im Allgemeinen 50 bis 260 °C, vorzugsweise 80 bis 220 °C.

Im Allgemeinen wird die Leichtsiederfraktion D1 in den POMDME-Reaktor (Schritt A)) zurückgeführt.

In einem Schritt E) wird die Schwersiederfraktion D2 in einen Phasentrennapparat eingespeist und in eine wässrige Phase E1 im Wesentlichen bestehend aus Formaldehyd, Wasser, Methylenglykol und Polyoxymethylenglykolen und eine organische Phase E2 enthaltend Tri- und Tetraoxymethylenglykoldimethylether (POMDME_{n=3,4}) aufgetrennt. Die organische Phase E2 enthält daneben ebenfalls noch Formaldehyd, Wasser, Methylenglykol und Polyoxymethylenglykole.

In einem Schritt F) wird die organische Phase E2 in eine dritte Destillationskolonne eingespeist und in eine Leichtsiederfraktion F1 im Wesentlichen bestehend aus Formaldehyd, Wasser, Methylenglykol und Polyoxymethylenglykolen und eine Schwersiederfraktion F2 im Wesentlichen bestehend aus Tri- und Tetraoxymethylenglykoldimethylether (POMDME_{n=3,4}) aufgetrennt.

Die dritte Destillationskolonne weist im Allgemeinen eine Stufenzahl von 1 bis 100, vorzugsweise von 1 bis 50 auf. Sie wird bei einem Druck von 0,1 bis 10 bar, vorzugsweise von 0,2 bis 6 bar, betrieben. Die Kopftemperatur beträgt im Allgemeinen 0 bis +160 °C, vorzugsweise 20 bis 130 °C, die Sumpftemperatur beträgt im Allgemeinen +100 bis +260 °C, vorzugsweise 150 bis 240 °C.

Die Schwersiederfraktion F2 stellt das Wertprodukt dar. Sie kann mehr als 99 Gew.-% POMDME_{n=3,4} enthalten.

Im Allgemeinen wird in einem weiteren (optionalen) Schritt G) die wässrigen Phase E1 weiter aufgearbeitet. Dazu wird diese in eine vierte Destillationskolonne eingespeist und in eine Leichtsiederfraktion G1 im Wesentlichen bestehend aus Formaldehyd, Wasser, Methylenglykol und Polyoxymethylenglykolen und eine Schwersiederfraktion im Wesentlichen bestehend aus Wasser aufgetrennt.

Die vierte Destillationskolonne weist im Allgemeinen eine Stufenzahl von 1 bis 30, vorzugsweise von 1 bis 20 auf. Sie wird bei einem Druck von 0,1 bis 10 bar, vorzugsweise von 0,2 bis 6 bar, betrieben. Die Kopftemperatur beträgt im Allgemeinen -20 bis +120 °C, vorzugsweise 20 bis 100 °C, die Sumpftemperatur beträgt im Allgemeinen +40 bis +180 °C, vorzugsweise 60 bis 150 °C.

Die Leichtsiederfraktionen F1 und/oder G1 können als Rückführströme in die zweite Destillationskolonne (Schritt D)) zurückgeführt werden. Bevorzugt werden sie in die zweite Destillationskolonne zurückgeführt. Die Leichtsiederfraktionen F1 und/oder G1 können aber auch als Rückführströme in den POMDME-Reaktor (Schritt A)) zurückgeführt werden.

Die Erfindung wird durch das nachstehende Beispiel näher erläutert.

### Beispiel

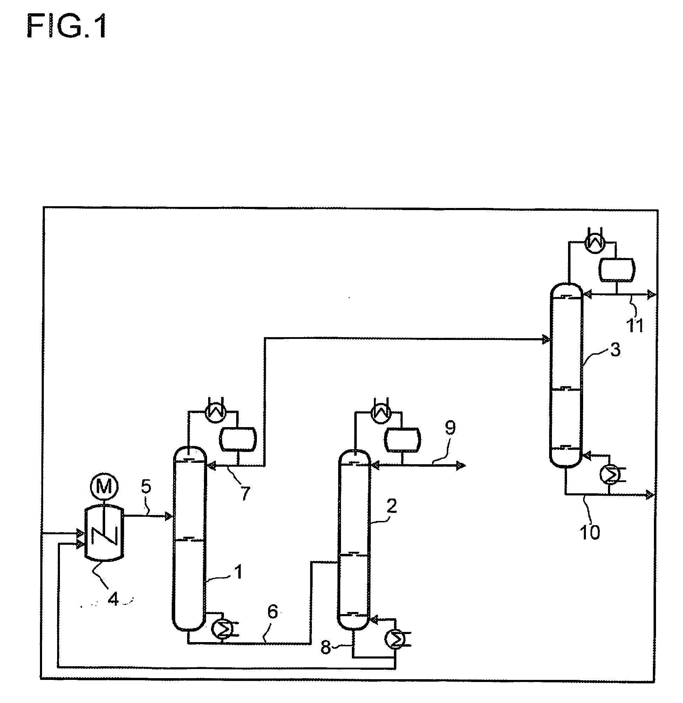

Bei der thermodynamischen Simulation des in Abbildung 1 dargestellten Verfahrensschemas wurden die in der Tabelle aufgeführten Stoffströme 6 - 11 am Sumpf beziehungsweise am Kopf der Kolonnen 1, 2 und 3 erhalten.

Es wurden folgende Parameter gewählt: Die Kolonne 1 wird bei einem Druck von 2 bar mit 40 theoretischen Stufen betrieben. Das Rücklaufverhältnis beträgt 1,5, die Kopftemperatur 77 °C und die Sumpftemperatur 145 °C. Der Zulauf 5 erfolgt auf dem 20. Boden der Kolonne 1.

Der Sumpfaustrag 6 der Kolonne 1 wird der Kolonne 2 auf dem 15. Boden zugeführt. Die Kolonne 2 enthält 30 Böden und wird bei einem Druck von 1,5 bar betrieben. Die Kopftemperatur beträgt 127 °C, die Sumpftemperatur beträgt 173 °C. Das Rücklaufverhältnis beträgt 1,5. Der Sumpfaustrag 8 der Kolonne 2 wird in den Reaktor 4 zurückgeführt.

Der Kopfaustrag 9 der Kolonne 1 wird der Kolonne 3 zugeführt. Dieser Strom wird auf dem 20. Boden zugeführt. Die Kolonne 3 hat insgesamt 30 Böden. Sie wird bei einem Druck von 1,7 bar betrieben. Das Rücklaufverhältnis beträgt 1,8. Die Kopftemperatur beträgt 59 °C, die Sumpftemperatur beträgt 92 °C.

Die Zusammensetzung der einzelnen Ströme ist in der nachstehenden Tabelle in Gew.-% angegeben.

| Strom | 5 | 6 | 7 | 8 | 9 | 10 | 11 |
|---|---|---|---|---|---|---|---|
| POMDMEn=3 | 4% | 21% | 0% | 98% | 0% | 0% | 0% |
| Formaldehyd | 37% | 0% | 46% | 0% | 0% | 66% | 0% |
| Trioxan | 15% | 79% | 0% | 2% | 100% | 0% | 0% |
| POMDMEn=2 | 17% | 0% | 21% | 0% | 0% | 30% | 0% |
| Methylal | 18% | 0% | 22% | 0% | 0% | 0% | 72% |
| Methanol | 6% | 0% | 7% | 0% | 0% | 0% | 24% |
| Methylformiat | 1% | 0% | 1% | 0% | 0% | 0% | 4% |
| Wasser | 2% | 0% | 3% | 0% | 0% | 4% | 0% |
| Menge [kg/h] | 100 | 19,0 | 81,0 | 4,1 | 14,9 | 55,9 | 25,1 |

## Patentansprüche

1. Verfahren zur Herstellung von Trioxan aus Trioxymethylenglykoldimethylether (POMDMEₙ₌₃) durch Umsetzung von Trioxymethylenglykoldimethylether in Gegenwart eines sauren Katalysators und anschließender destillativer Aufarbeitung des Reaktionsgemischs mit den Schritten:
a) Einspeisung von Trioxymethylenglykoldimethylether (POMDMEₙ₌₃) oder eines Trioxymethylenglykoldimethylether enthaltenden Gemischs in einen Reaktor und Umsetzung in Gegenwart eines sauren Katalysators zu einem Gemisch a enthaltend Trioxan, Formaldehyd, Wasser, Methylenglykol (MG), Polyoxymethylenglykole (MG_{n>1}), Methanol, Hemiformale (HF), Methylal (POMDMEₙ₌₁) und Polyoxymethylenglykoldimethylether (POMDME_{n>1});
b) destillative Auftrennung des Reaktionsgemisches a in eine Leichtsiederfraktion b1 enthaltend Formaldehyd, Wasser, Methylenglykol, Methanol, Hemiformal (HFₙ₌₁), Methylal und Dioxymethylenglykoldimethylether (POMDMEₙ₌₂) und eine Schwersiederfraktion b2 enthaltend Trioxan, Polyoxymethylenglykole (MG_{n>1}), Hemiformale (HF_{n>1}) und Polyoxymethylenglykoldimethylether (POMDME_{n>2});
c) destillative Auftrennung der Schwersiederfraktion b2 in eine Leichtsiederfraktion c1 enthaltend Trioxan und eine Schwersiederfraktion c2 enthaltend Polyoxymethylenglykole (MG_{n>1}), Hemiformale (HF_{n>1}) und Polyoxymethylenglykoldimethylether (POMDME_{n>2)}.

2. Verfahren nach Anspruch 1, **dadurch gekennzeichnet, dass** die Schwersiederfraktion c2 in den Reaktor des Schrittes a) zurückgeführt wird.

3. Verfahren nach Anspruch 2, **dadurch gekennzeichnet, dass** die Leichtsiederfraktion b1 in eine Leichtsiederfraktion d1 enthaltend Wasser, Methylal, Methylenglykol, Methanol, Methylformiat und Hemiformal (HFₙ₌₁) und eine Schwersiederfraktion d2 enthaltend Formaldehyd, Wasser und Dioxymethylenglykoldimethylether (POMDMEₙ₌₂) aufgetrennt wird, wobei die Schwersiederfraktion d2 in den Reaktor des Schrittes a) zurückgeführt wird.

4. Verfahren nach einem der Ansprüche 1 bis 3, **dadurch gekennzeichnet, dass** der Trioxymethylenglykoldimethylether (POMDMEₙ₌₃) oder das diesen enthaltende Gemisch in einer vorgelagerten Synthese durch Umsetzung eines Formaldehyd und Methanol enthaltenden Gemischs und anschließender destillativer Aufarbeitung des Produktgemischs erhalten wird.

5. Verfahren nach Anspruch 4, **dadurch gekennzeichnet, dass** die Leichtsiederfraktion b1 enthaltend Formaldehyd, Wasser, Methylenglykol, Methanol, Hemiformal (HFₙ₌₁), Methylal und Dioxymethylenglykoldimethylether (POMDMEₙ₌₂) in die Trioxymethylenglykoldimethylether-Synthese zurückgeführt wird.

6. Verfahren nach Anspruch 3, **dadurch gekennzeichnet, dass** die Leichtsiederfraktion d1 in die Trioxymethylenglykoldimethylether-Synthese zurückgeführt wird.

7. Verfahren nach einem der Ansprüche 1 bis 6, **dadurch gekennzeichnet, dass** in Schritt a) ein Gemisch enthaltend Tri- und Tetraoxymethylenglykoldimethylether (POMDME_{n=3,4}) eingesetzt wird.

## Claims

1. A process for preparing trioxane from trioxymethylene glycol dimethyl ether (POMDMEₙ₌₃) by converting trioxymethylene glycol dimethyl ether in the presence of an acidic catalyst and subsequent distillative workup of the reaction mixture, comprising the steps of:
a) feeding trioxymethylene glycol dimethyl ether (POMDMEₙ₌₃) or a mixture comprising trioxymethylene glycol dimethyl ether into a reactor and converting it in the presence of an acidic catalyst to a mixture a comprising trioxane, formaldehyde, water, methylene glycol (MG), polyoxymethylene glycols (MG_{n>1}), methanol, hemiformals (HF), methylal (POMDMEₙ₌₁) and polyoxymethylene glycol dimethyl ethers (POMDME_{n>1}) ;
b) distillatively separating the reaction mixture a into a low boiler fraction b1 comprising formaldehyde, water, methylene glycol, methanol, hemiformal (HFₙ₌₁), methylal and dioxymethylene glycol dimethyl ether (POMDMEₙ₌₂), and a high boiler fraction b2 comprising trioxane, polyoxymethylene glycols (MG_{n>1}), hemiformals (HF_{n>1}) and polyoxymethylene glycol dimethyl ethers (POMDME_{n>2});
c) distillatively separating the high boiler fraction b2 into a low boiler fraction c1 comprising trioxane, and a high boiler fraction c2 comprising polyoxymethylene glycols (MG_{n>1}) hemiformals (HF_{n>1}) and polyoxymethylene glycol dimethyl ethers (POMDME_{n>2}).

2. The process according to claim 1, wherein the high boiler fraction c2 is recycled into the reactor of step a).

3. The process according to claim 2, wherein the low boiler fraction b1 is separated into a low boiler fraction d1 comprising water, methylal, methylene glycol, methanol, methyl formate and hemiformal (HFₙ₌₁), and a high boiler fraction d2 comprising formaldehyde, water and dioxymethylene glycol dimethyl ether (POMDMEₙ₌₂), the high boiler fraction d2 being recycled into the reactor of step a).

4. The process according to any of claims 1 to 3, wherein the trioxymethylene glycol dimethyl ether (POMDMEₙ₌₃) or the mixture comprising it is obtained in a preceding synthesis by converting a mixture comprising formaldehyde and methanol and subsequently working-up the product mixture by distillation.

5. The process according to claim 4, wherein the low boiler fraction b1 comprising formaldehyde, water, methylene glycol, methanol, hemiformal (HFₙ₌₁), methylal and dioxymethylene glycol dimethyl ether (POMDMEₙ₌₂) is recycled into the trioxymethylene glycol dimethyl ether synthesis.

6. The process according to claim 3, wherein the low boiler fraction d1 is recycled into the trioxymethylene glycol dimethyl ether synthesis.

7. The process according to any of claims 1 to 6, wherein a mixture comprising tri- and tetraoxymethylene glycol dimethyl ether (POYDME_{n=3,4}) is used in step a).

## Revendications

1. Procédé de fabrication de trioxane à partir d'éther diméthylique de trioxyméthylène glycol (POMDMEₙ₌₃) par réaction d'éther diméthylique de trioxyméthylène glycol en présence d'un catalyseur acide, puis traitement par distillation du mélange réactionnel selon les étapes :
a) introduction d'éther diméthylique de trioxyméthylène glycol (POMDMEₙ₌₃) ou d'un mélange contenant de l'éther diméthylique de trioxyméthylène glycol dans un réacteur et réaction en présence d'un catalyseur acide pour former un mélange a contenant du trioxane, du formaldéhyde, de l'eau, du méthylène glycol (MG), des polyoxyméthylène glycols (MG_{n>1}), du méthanol, des hémiformals (HF), du méthylal (POMDMEₙ₌₁) et des éthers diméthyliques de polyoxyméthylène glycol (POMDMEₙ₋₁) ;
b) séparation par distillation du mélange réactionnel a en une fraction de point d'ébullition bas b1 contenant du formaldéhyde, de l'eau, du méthylène glycol, du méthanol, des hémiformals (HFₙ₌₁), du méthylal et de l'éther diméthylique de dioxyméthylène glycol (POMDMEₙ₌₂) et une fraction de point d'ébullition élevé b2 contenant du trioxane, des polyoxyméthylène glycols (MG_{n>1}), des hémiformals (HF_{n>1}) et des éthers diméthyliques de polyoxyméthylène glycol (POMDME_{n>2}) ;
c) séparation par distillation de la fraction de point d'ébullition élevé b2 en une fraction de point d'ébullition bas c1 contenant du trioxane et une fraction de point d'ébullition élevé c2 contenant des polyoxyméthylène glycols (MG_{n>1}) , des hémiformals (HF_{n>1}) et des éthers diméthyliques de polyoxyméthylène glycol (POMDME_{n>2}).

2. Procédé selon la revendication 1, **caractérisé en ce que** la fraction de point d'ébullition élevé c2 est recyclée dans le réacteur de l'étape a).

3. Procédé selon la revendication 2, **caractérisé en ce que** la fraction de point d'ébullition bas b1 est séparée en une fraction de point d'ébullition bas d1 contenant de l'eau, du méthylal, du méthylène glycol, du méthanol, du formiate de méthyle et de l'hémiformal (HFₙ₌₁) et une fraction de point d'ébullition élevé d2 contenant du formaldéhyde, de l'eau et de l'éther diméthylique de dioxyméthylène glycol (POMDMEₙ₌₂), la fraction de point d'ébullition élevé d2 étant recyclée dans le réacteur de l'étape a).

4. Procédé selon l'une quelconque des revendications 1 à 3, **caractérisé en ce que** l'éther diméthylique de trioxyméthylène glycol (POMDMEₙ₌₃) ou le mélange contenant celui-ci est obtenu lors d'une synthèse située en amont par réaction d'un mélange contenant du formaldéhyde et du méthanol, puis traitement par distillation du mélange de produits.

5. Procédé selon la revendication 4, **caractérisé en ce que** la fraction de point d'ébullition bas b1 contenant du formaldéhyde, de l'eau, du méthylène glycol, du méthanol, des hémiformals (HFₙ₌₁), du méthylal et de l'éther diméthylique de dioxyméthylène glycol (POMDMEₙ₌₂) est recyclée dans la synthèse de l'éther diméthylique de trioxyméthylène glycol.

6. Procédé selon la revendication 3, **caractérisé en ce que** la fraction de point d'ébullition bas d1 est recyclée dans la synthèse de l'éther diméthylique de trioxyméthylène glycol.

7. Procédé selon l'une quelconque des revendications 1 à 6, **caractérisé en ce qu'**un mélange contenant des éthers diméthyliques de tri- et tétraoxyméthylène glycol (POMDME_{n=3,4}) est utilisé à l'étape a).
